# EUROPEAN PATENT SPECIFICATION

(11) **EP 2 897 971 B1**
(45) Date of publication and mention of the grant of the patent: **31.05.2017**
(21) Application number: 13766034.6
(22) Date of filing: 20.09.2013
(51) Int. Cl.: C07K 1/12, C12P 21/02, C07K 7/50, C12N 9/52

(54) **METHOD FOR PRODUCING A CYCLIC PEPTIDE**
VERFAHREN ZUR HERSTELLUNG EINES ZYKLISCHEN PEPTIDS
PROCÉDÉ DE PRODUCTION D'UN PEPTIDE CYCLIQUE

(30) Priority: 24.09.2012 EP 12185649
(43) Date of publication of application: 29.07.2015
(73) Proprietor: DSM Sinochem Pharmaceuticals Netherlands B.V., 2613 AX Delft (NL)
(72) Inventor: LAAN, VAN DER, Jan Metske, NL-6100 AA Echt (NL); KERKMAN, Richard, NL-6100 AA Echt (NL); LUITEN, Rudolf, Gijsbertus, Marie, NL-6100 AA Echt (NL); BERG, VAN DEN, Marco Alexander, NL-6100 AA Echt (NL)
(74) Representative: van Dam, Vincent
(86) International application number: PCT/EP2013/069587
(87) International publication number: WO 2014/044803

(56) References cited:
- WO-A1-01/05815
- WO-A1-2006/063783
- SHOJI J ET AL: "The amino acid sequence of cerexin A (studies on antibiotics from the genus Bacillus. VII.", THE JOURNAL OF ANTIBIOTICS OCT 1975, vol. 28, no. 10, October 1975 (1975-10), pages 764-769, XP55089971, ISSN: 0021-8820
- YUKIO KIMURA ET AL: "Polymyxin Acylase: a NewEnzymefor Preparing Starting Materials for Semisynthetic Polymyxin Antibiotics", AGRICULTURAL AND BIOLOGICAL CHEMISTRY, vol. 51, no. 6, 1 January 1987 (1987-01-01), pages 1617-1623, XP055089976, DOI: 10.1271/bbb1961.51.1617
- KIMURA E A: "Polymixin acylase: purification and characterization, with special reference to broad substrate specificity", AGRICULTURAL AND BIOLOGICAL CHEMISTRY, JAPAN SOC. FOR BIOSCIENCE, BIOTECHNOLOGY AND AGROCHEM, TOKYO, JP, vol. 53, no. 2, 1 January 1989 (1989-01-01), pages 497-504, XP002149548, ISSN: 0002-1369
- S.-Y. PARK ET AL: "Identification of Extracellular N-Acylhomoserine Lactone Acylase from a Streptomyces sp. and Its Application to Quorum Quenching", APPLIED AND ENVIRONMENTAL MICROBIOLOGY, vol. 71, no. 5, 1 May 2005 (2005-05-01), pages 2632-2641, XP055089940, ISSN: 0099-2240, DOI: 10.1128/AEM.71.5.2632-2641.2005
- CHEN CHIN-NUNG ET AL: "A probable aculeacin A acylase from the Ralstonia solanacearum GMI1000 is N-acyl-homoserine lactone acylase with quorum-quenching activity", BMC MICROBIOLOGY, BIOMED CENTRAL LTD, GB, vol. 9, no. 1, 9 May 2009 (2009-05-09), page 89, XP021048261, ISSN: 1471-2180, DOI: 10.1186/1471-2180-9-89
- M. BOKHOVE ET AL: "The quorum-quenching N-acyl homoserine lactone acylase PvdQ is an Ntn-hydrolase with an unusual substrate-binding pocket", PROCEEDINGS OF THE NATIONAL ACADEMY OF SCIENCES, vol. 107, no. 2, 12 January 2010 (2010-01-12), pages 686-691, XP055090606, ISSN: 0027-8424, DOI: 10.1073/pnas.0911839107

### SEQUENCE LISTING

<110> DSM Sinochem Pharmaceuticals Netherlands B.V.
<120> Method for producing a cyclic peptide
<130> 29068-WO-PCT
<150> EP12185649.6 <151> 2012-09-24
<160> 6
<170> BiSSAP 1.2
<210> 1
   <211> 762
   <212> PRT
   <213> Pseudomonas aeruginosa
<220>
   <223> wild-type acylase from P. aeruginosa PA01
<400> 1
   Met Gly Met Arg Thr Val Leu Thr Gly Leu Ala Gly Met Leu Leu Gly
   1               5                   10                  15
   Ser Met Met Pro Val Gln Ala Asp Met Pro Arg Pro Thr Gly Leu Ala
               20                  25                  30
   Ala Asp Ile Arg Trp Thr Ala Tyr Gly Val Pro His Ile Arg Ala Lys
           35                  40                  45
   Asp Glu Arg Gly Leu Gly Tyr Gly Ile Gly Tyr Ala Tyr Ala Arg Asp
       50                  55                  60
   Asn Ala Cys Leu Leu Ala Glu Glu Ile Val Thr Ala Arg Gly Glu Arg
   65                  70                  75                  80
   Ala Arg Tyr Phe Gly Ser Glu Gly Lys Ser Ser Ala Glu Leu Asp Asn
                   85                  90                  95
   Leu Pro Ser Asp Ile Phe Tyr Ala Trp Leu Asn Gln Pro Glu Ala Leu
               100                 105                 110
   Gln Ala Phe Trp Gln Ala Gln Thr Pro Ala Val Arg Gln Leu Leu Glu
           115                 120                 125
   Gly Tyr Ala Ala Gly Phe Asn Arg Phe Leu Arg Glu Ala Asp Gly Lys
       130                 135                 140
   Thr Thr Ser Cys Leu Gly Gln Pro Trp Leu Arg Ala Ile Ala Thr Asp
   145                 150                 155                 160
   Asp Leu Leu Arg Leu Thr Arg Arg Leu Leu Val Glu Gly Gly Val Gly
                   165                 170                 175
   Gln Phe Ala Asp Ala Leu Val Ala Ala Ala Pro Pro Gly Ala Glu Lys
               180                 185                 190
   Val Ala Leu Ser Gly Glu Gln Ala Phe Gln Val Ala Glu Gln Arg Arg
           195                 200                 205
   Gln Arg Phe Arg Leu Glu Arg Gly Ser Asn Ala Ile Ala Val Gly Ser
       210                 215                 220
   Glu Arg Ser Ala Asp Gly Lys Gly Met Leu Leu Ala Asn Pro His Phe
   225                 230                 235                 240
   Pro Trp Asn Gly Ala Met Arg Phe Tyr Gln Met His Leu Thr Ile Pro
                   245                 250                 255
   Gly Arg Leu Asp Val Met Gly Ala Ser Leu Pro Gly Leu Pro Val Val
               260                 265                 270
   Asn Ile Gly Phe Ser Arg His Leu Ala Trp Thr His Thr Val Asp Thr
           275                 280                 285
   Ser Ser His Phe Thr Leu Tyr Arg Leu Ala Leu Asp Pro Lys Asp Pro
       290                 295                 300
   Arg Arg Tyr Leu Val Asp Gly Arg Ser Leu Pro Leu Glu Glu Lys Ser
   305                 310                 315                 320
   Val Ala Ile Glu Val Arg Gly Ala Asp Gly Lys Leu Ser Arg Val Glu
                   325                 330                 335
   His Lys Val Tyr Gln Ser Ile Tyr Gly Pro Leu Val Val Trp Pro Gly
               340                 345                 350
   Lys Leu Asp Trp Asn Arg Ser Glu Ala Tyr Ala Leu Arg Asp Ala Asn
           355                 360                 365
   Leu Glu Asn Thr Arg Val Leu Gln Gln Trp Tyr Ser Ile Asn Gln Ala
       370                 375                 380
   Ser Asp Val Ala Asp Leu Arg Arg Arg Val Glu Ala Leu Gln Gly Ile
   385                 390                 395                 400
   Pro Trp Val Asn Thr Leu Ala Ala Asp Glu Gln Gly Asn Ala Leu Tyr
                   405                 410                 415
   Met Asn Gln Ser Val Val Pro Tyr Leu Lys Pro Glu Leu Ile Pro Ala
               420                 425                 430
   Cys Ala Ile Pro Gln Leu Val Ala Glu Gly Leu Pro Ala Leu Gln Gly
           435                 440                 445
   Gln Asp Ser Arg Cys Ala Trp Ser Arg Asp Pro Ala Ala Ala Gln Ala
       450                 455                 460
   Gly Ile Thr Pro Ala Ala Gln Leu Pro Val Leu Leu Arg Arg Asp Phe
   465                 470                 475                 480
   Val Gln Asn Ser Asn Asp Ser Ala Trp Leu Thr Asn Pro Ala Ser Pro
                   485                 490                 495
   Leu Gln Gly Phe Ser Pro Leu Val Ser Gln Glu Lys Pro Ile Gly Pro
               500                 505                 510
   Arg Ala Arg Tyr Ala Leu Ser Arg Leu Gln Gly Lys Gln Pro Leu Glu
           515                 520                 525
   Ala Lys Thr Leu Glu Glu Met Val Thr Ala Asn His Val Phe Ser Ala
       530                 535                 540
   Asp Gln Val Leu Pro Asp Leu Leu Arg Leu Cys Arg Asp Asn Gln Gly
   545                 550                 555                 560
   Glu Lys Ser Leu Ala Arg Ala Cys Ala Ala Leu Ala Gln Trp Asp Arg
                   565                 570                 575
   Gly Ala Asn Leu Asp Ser Gly Ser Gly Phe Val Tyr Phe Gln Arg Phe
               580                 585                 590
   Met Gln Arg Phe Ala Glu Leu Asp Gly Ala Trp Lys Glu Pro Phe Asp
           595                 600                 605
   Ala Gln Arg Pro Leu Asp Thr Pro Gln Gly Ile Ala Leu Asp Arg Pro
       610                 615                 620
   Gln Val Ala Thr Gln Val Arg Gln Ala Leu Ala Asp Ala Ala Ala Glu
   625                 630                 635                 640
   Val Glu Lys Ser Gly Ile Pro Asp Gly Ala Arg Trp Gly Asp Leu Gln
                   645                 650                 655
   Val Ser Thr Arg Gly Gln Glu Arg Ile Ala Ile Pro Gly Gly Asp Gly
               660                 665                 670
   His Phe Gly Val Tyr Asn Ala Ile Gln Ser Val Arg Lys Gly Asp His
           675                 680                 685
   Leu Glu Val Val Gly Gly Thr Ser Tyr Ile Gln Leu Val Thr Phe Pro
       690                 695                 700
   Glu Glu Gly Pro Lys Ala Arg Gly Leu Leu Ala Phe Ser Gln Ser Ser
   705                 710                 715                 720
   Asp Pro Arg Ser Pro His Tyr Arg Asp Gln Thr Glu Leu Phe Ser Arg
                   725                 730                 735
   Gln Gln Trp Gln Thr Leu Pro Phe Ser Asp Arg Gln Ile Asp Ala Asp
               740                 745                 750
   Pro Gln Leu Gln Arg Leu Ser Ile Arg Glu
           755                 760
<210> 2
   <211> 2289
   <212> DNA
   <213> Pseudomonas aeruginosa
<220>
   <221> source
   <222> 1..2289
   <223> /organism="Pseudomonas aeruginosa" /note="gene encoding the acylase from Pseudomonas aeruginosa PA01" /mol_type="unassigned DNA"
<400> 2
   atggggatgc gtaccgtact gaccggcctg gccggcatgc tgttgggttc gatgatgccg 60
   gtccaggccg atatgccgcg gccgaccggg ctggccgcgg atatccgctg gaccgcctat 120
   ggcgtgccgc acatccgggc caaggatgag cgcggcctgg gctatggcat cggctacgcc 180
   tacgcgcgcg acaacgcctg cctgctggcc gaggagatcg tcaccgcgcg cggcgagcgg 240
   gcgcgctatt tcggcagcga gggcaagtcg tcggccgagc tggacaacct gccgtccgac 300
   atcttctacg cctggctcaa ccaacccgag gcgctgcaag ccttctggca ggcgcagacg 360
   cccgcggtac gccagttgct cgaaggctac gccgccggtt tcaaccgctt cctccgcgag 420
   gccgacggca agaccaccag ttgccttggc cagccctggc tgcgggccat cgcgaccgat 480
   gacctgctgc gcctgacccg gcgcctgctg gtcgaaggcg gggtcggcca gttcgccgac 540
   gcgctggtgg ccgccgcgcc gcccggagcg gagaaggtcg ccttgagcgg cgagcaggcg 600
   ttccaggtcg ccgagcagcg gcgccagcgc ttccgcctgg agcgcggcag caacgccatt 660
   gccgttggca gcgaacgttc ggcggacggc aagggcatgc tcctggccaa cccgcacttc 720
   ccctggaacg gcgcgatgcg tttctaccag atgcacctga ccattcccgg ccggctcgac 780
   gtgatggggg cctcgctgcc cggcctgccg gtggtcaaca tcggcttcag ccgccacctg 840
   gcctggaccc acacggtgga tacctccagc cacttcaccc tgtatcgcct ggcgctggac 900
   ccgaaggacc cgcggcgcta cctggttgac ggtcgttcgc tgccgctgga ggagaagtcc 960
   gtcgcgatcg aggtgcgcgg cgccgacggc aagctgtcgc gcgtcgagca caaggtctac 1020
   cagtcgatct acggcccgct ggtggtctgg cccggcaagc tggactggaa ccgcagcgag 1080
   gcctatgcgc tgcgtgacgc caacctggag aacactcggg tactgcaaca gtggtactcg 1140
   atcaaccagg ccagcgacgt cgccgacctg cgccggcgcg tcgaggcgct acagggtatc 1200
   ccctgggtca acaccctggc cgcggacgag cagggcaacg ccctgtacat gaaccagtcg 1260
   gtggtgccct acctgaagcc ggaactgatc cccgcctgcg ccattccgca actggtcgcc 1320
   gaaggcctgc cggccctcca ggggcaggac agccgctgtg cctggagtcg cgacccggcc 1380
   gcggcccagg ctggcatcac cccggcggcg caactgccgg tgctgttgcg cagggacttc 1440
   gtgcagaact ccaacgacag cgcctggctg accaacccgg cgagcccgct gcaaggcttc 1500
   tcgcccctgg tcagccagga gaagcccatc ggtccgcggg cgcgctacgc cctgagccgg 1560
   ctacagggca agcagccgct ggaggcgaag acgctggagg agatggtcac cgccaaccat 1620
   gtcttcagcg ccgaccaggt gctgccggac ctgctccgcc tgtgccgcga caaccagggc    1680
   gagaagtccc ttgcccgcgc ctgcgcggcc ctggcgcagt gggaccgtgg cgccaacctc    1740
   gacagcggca gcggcttcgt ctacttccag cgcttcatgc aacgcttcgc cgaactcgac    1800
   ggcgcgtgga aggaaccgtt cgatgcgcaa cgtcccctgg atacgccgca aggcatcgcc    1860
   ctcgaccggc cgcaggtggc gacccaggtg cgccaggcgc tggcggacgc ggcggcggag    1920
   gtggagaaga gcgggattcc cgacggcgcg cgctggggcg acctgcaagt gagcacccgt    1980
   ggccaggaac gcatcgcgat tcccggcggc gatggccatt tcggggtcta caacgcgatc    2040
   cagagcgtcc gcaagggcga ccacctggag gtggtcggcg gcactagcta catccagctg    2100
   gtgaccttcc ccgaggaagg tcctaaggct cgcgggttgc tggctttctc ccagtccagc    2160
   gatccgcgct cgccgcacta ccgcgaccag accgagctgt tttcccgcca gcaatggcag    2220
   accttgccgt tcagcgacag gcagatcgac gccgacccgc aactgcaacg gctaagcatt    2280
   cgcgaatga                                                             2289
<210> 3
   <211> 14
   <212> PRT
   <213> Pseudomonas
<220>
   <223> motif i)
<220>
   <221> VARIANT
   <222> join(2..5,7..8,11..13)
   <223> may be replaced with any amino acid
<220>
   <221> VARIANT
   <222> 6
   <223> may be replaced with Val
<220>
   <221> VARIANT
   <222> 10
   <223> may be replaced with Met
<400> 3
   Asp Leu Leu Arg Leu Thr Arg Arg Leu Leu Val Glu Gly Gly
   1               5                   10
<210> 4
   <211> 8
   <212> PRT
   <213> Pseudomonas
<220>
   <223> motif ii)
<220>
   <221> VARIANT
   <222> 1
   <223> may be replaced with Ile
<220>
   <221> VARIANT
   <222> 6..7
   <223> may be replaced with any amino acid
<400> 4
   Leu Pro Gly Leu Pro Val Val Asn
   1               5
<210> 5
   <211> 6
   <212> PRT
   <213> Pseudomonas
<220>
   <223> motif iii)
<220>
   <221> VARIANT
   <222> 4..5
   <223> may be replaced with any amino acid
<400> 5
   Arg Val Leu Gln Gln Trp
   1               5
<210> 6
   <211> 8
   <212> PRT
   <213> Pseudomonas
<220>
   <223> motif iv)
<220>
   <221> VARIANT
   <222> 5..7
   <223> may be replaced with any amino acid
<400> 6
   His Thr Val Asp Thr Ser Ser His
   1               5

## Description

### Field of the invention

The present invention relates to a method for producing a cyclic peptide compound or a salt thereof by deacylating the acyl chain of a cyclic lipopeptide by means of an acylase.

### Background of the invention

Cyclic peptides or cyclopeptides are polypeptides in which the terminal amine and carboxyl groups form an internal peptide bond. Several cyclic peptides are known for their advantageous medicinal properties. In nature cyclic peptides can range from just a few amino acids in length to hundreds. Cyclic peptides can be naturally occurring compounds but may also be obtained by total synthesis or by synthetic or genetic modification of naturally occurring or naturally produced precursors. The latter class is referred to as semi synthetic cyclic peptides. When cyclic peptides bear one or more lipid tails or acyl chains, such cyclic peptides are referred to as cyclic lipopeptides. Cyclic lipopeptides with antibiotic activity include compounds such as daptomycin and amphomycin and an excellent example of cyclic lipopeptides is the class of echinocandins which are potent antifungals. Echinocandins are amphiphilic hexapeptides with an N-linked acyl lipid side chain and a molecular weight of approximately 1200 Da. Examples of medicinally useful echinocandins are the cyclic hexapeptides anidulafungin, caspofungin, cilofungin and micafungin which are useful in treating fungal infections, especially those caused by *Aspergillus*, *Blastomyces*, *Candida*, *Coccidioides* and *Histoplasma.* Anidulafungin (1-[(4*R*,5*R*)-4,5-dihydroxy-*N*2-[[4"-(pentyloxy)[1,1':4',1"-terphenyl]-4-yl]carbonyl]-L-ornithine]echinocandin B), caspofungin (1-[(4*R*,5*S*)-5-[(2-aminoethyl)amino]-*N*2-(10,12-dimethyl-1-oxotetradecyl)-4-hydroxy-L-ornithine]-5-[(3*R*)-3-hydroxy-L-ornithine]-pneumocandin B₀) and micafungin (1-[(4*R*,5*R*)-4,5-dihydroxy-*N*2-[4-[5-[4-(pentyloxy)phenyl]-3-isoxazolyl]benzoyl]-L-ornithine]-4-[(4S)-4-hydroxy-4-[4-hydroxy-3-(sulfooxy)phenyl]-L-threonine]pneumocandin A₀) are all semi synthetic cyclic hexapeptides derivable from naturally occurring echinocandins such as for instance echinocandin B, pneumocandin A₀, pneumocandin B₀ or FR 901379.

Natural cyclic lipopeptides are typically produced by micro-organisms. Daptomycin is produced by the soil bacterium *Streptomyces roseosporus.* Amphomycin is produced by *Streptomyces canus.* Natural echinocandins such as echinocandin B, echinocandin C, aculeacin Ay, pneumocandin B₀ and FR 901379 are also typically produced by various micro-organisms. For example, echinocandin B is produced by the fungus *Aspergillus nidulans* and FR 901379 is produced by the fungus *Coleophoma empetri.*

The acyl chain of cyclic lipopeptides has shown to be an important determinant of antifungal activity and toxicity (Debono M. & Gordee R.S., Annu. Rev. Microbiol. 48, 471 (1994)). For instance the naturally occurring cyclic antifungal lipopeptide FR 901379 bearing a fatty acid acyl group attached to the N-terminus shows potent *in vivo* antifungal activity (Iwamoto, T., Fujie A., Nitta, K., Hashimoto, S., Okuhara, M., Kohsaka, M., J. Antibiot. 47, 1092 (1994). Unfortunately, just like some other naturally occurring echinocandins it also shows high hemolytic activity. Enzymatic removal of the fatty acid chain and replacement for an octyloxybenzoyl acyl chain showed that the original activity of FR 901379 was retained but that hemolytic activity was significantly reduced. Another example is micafungin, which is produced by exchanging the lipid tail of FR 901379 for a complex 3,5-diphenyl substituted isoxazole acyl group (Fujie, Pure Appl. Chem. 79, No. 4, pp.603-614 (2007)). Deacylation of the natural acyl chain of cyclic lipopeptides thus allows for the introduction of alternative side chains which improve antifungal efficacy and decrease hemolytic activity.

Deacylation of cyclic lipopeptides, such as echinocandins, has been established by means of the Aculeacin A acylase from *Actinoplanes utahensis.* JP 4228072(A) discloses an enzyme that catalyzes the deacylation of the lipid acyl portion of lipid cyclic peptide metabolites such as echinocandin B and aculeacin. JP 4075585 describes that this acylase can be cultured in *Streptomyces* as host organism. After Aculeacin A acylase is collected from a culture solution it is directly used to deacylate a substrate. In the course of optimizing the enzymatic deacylation it was discovered that some acylases of *Streptomyces* spp. were more efficient. EP 0885957 A1 describes cyclic lipopeptide acylases from the genus *Streptomyces* which are capable of deacylating the acyl chain of a cyclic lipopeptide compound, e.g. the abovementioned echinocandin FR 901379 or analogs thereof and a method of producing a cyclic peptide compound which comprises using said acylases. Polymyxin acylase used to deacylate the macrocyclic polymyxin E has been described in Shoji et al., 1975, Journal of Antibiotics 28, 764-769, Kimura et al., 1987, Agricultural Biological Chemistry 51, 1617-1623 and Kimura et al., 1989, Agricultural Biological Chemistry 53, 497-504. Application of polymyxin acylase in the deacylation of macrocyclic lipopeptides has been described in WO 01/05815 and WO 2006/063783. Also certain N-acylhomoserine lactones have enzymatic activity against macrocyclic lipopeptides as reported in Park et al., 2005, Applied Environmental Microbiology 71, 2632-2641 and in Chen Chin-Nung et al., 2009, BMC Microbiology 9, 89-99.

The disadvantage of the above described methods for deacylation of cyclic lipopeptides is that it is not possible to produce the abovementioned acylases in a functional form and in a sufficient amount in an industrially preferred production host such as *Escherichia coli* or *Bacillus.* Production of acylase in *Streptomyces* or *Actinoplanes utahensis* is not preferred in an industrial setting because it leads to relatively low enzyme yields because of low expression levels in these organisms and because these organisms are less suitable for use in fermenter systems. For instance, use of *Streptomyces lividans* in fermenter systems often leads to problems with respect to viscosity which prevents that high cell densities are obtained. Furthermore, there is a problem with respect to downstream processing when abovementioned acylases are produced in *Streptomyces* or *Actinoplanes utahensis.* In these organisms the enzyme seems to be attached to the biomass and in the purification process of the acylase enzyme extractions with high salt concentrations are required (EP0885957 A1, Kreuzman et al., 2000, Journal of Industrial Microbiology & Biotechnology, 24, 173-180, Ueda et al., 2011, Journal of Bioscience and Bioengineering 112, 409-414 Ueda et al., 2011, Journal of Antibiotics 64, 169-175). The use of salts to release an enzyme from the broth is undesired at large scale industrial production with respect to corrosion and environmental issues.

In view of the strict regulatory and health related requirements there remains a need for improved purification and isolation methods. Therefore the present invention aims to improve deacylation of cyclic lipopeptides on industrial scale by providing a method that applies an acylase that is producible in a functional form in an industrially preferred production host.

### Detailed description of the invention

The present invention relates to a method for producing a cyclic peptide compound or a salt thereof which comprises contacting a cyclic lipopeptide compound or a salt thereof with an acylase derived from a microorganism belonging to the genus *Pseudomonas* to deacylate the lipid acyl moiety of said cyclic lipopeptide. By using a *Pseudomonas* acylase in a method of producing cyclic peptides from cyclic lipopeptides it becomes possible to produce a cyclic peptide from a cyclic lipopeptide in a more efficient way because the *Pseudomonas* acylase can be produced in an efficient and large scale manner in an industrially preferred production organism. Accordingly the invention provides an important improvement in the production process of antibiotic cyclic peptides.

Following deacylation the obtained cyclic peptide may be subjected to an additional step of reacylating said cyclic peptide resulting in a reacylated cyclic peptide. This way a desired antibiotic product can be obtained. Reacylating the cyclic peptide usually involves a process of synthesizing the new acyl chain for the cyclic peptide, followed by a process of coupling the acyl chain to the free amino group of the cyclic peptide. After reacylation, the acyl chain of the reacylated cyclic peptide may be further processed resulting in a further processed reacylated cyclic peptide. This can be done by any suitable chemical, semi-chemical or enzymatic method. The process of reacylating is usually followed by purification of the thus obtained new cyclic lipopeptide to the right quality and/or crystal form.

A process of synthesizing a new acyl chain may be any method that is known to the skilled person such as total chemical synthesis or semi-synthesis, i.e. fermentation followed by one or more chemical conversions. One example of a process of reacylation of a cyclic peptide may be the chemical synthesis of an acyl chain from 1-bromooctane and 4-hydroxybenzoic acid followed by the coupling of the resulting 2,4,5-trichlorophenyl 4-(n-octyloxy)benzoate to a free amino group of a deacylated derivative of FR 901379 as described in Fujie, Pure Appl. Chem. 79, No. 4, pp.603-614 (2007).

The process of production of a cyclic lipopeptide according to the invention may for instance result in micafungin or a salt thereof. The reacylated cyclic peptide or further processed reacylated cyclic peptide described above may thus be micafungin or a salt thereof. The process of producing micafungin starts with production of FR 901379. This may be realized by fermentation by *Coleophoma empetri* followed by downstream processing to obtain the required purity of FR 901379 or a salt thereof.

In order to obtain micafungin, first the acyl side chain of FR 901379 has to be removed. For this purpose expression of the acylase from *Pseudomonas* in an industrially preferred production strain is realized, followed by downstream processing and formulation of the enzyme. This is followed by the biocatalytic process in which the acyl side chain of FR 901379 is removed leading to a micafungin precursor.

In order to synthesize micafungin from its precursor first an appropriate side chain must be available. For micafungin this is a 3,5-diphenyl-substituted isoxazole. Side chains may be synthesized using techniques that are known to the skilled person. The side chain is subsequently coupled to the micafungin precursor by regular chemical, semi chemical or enzymatic methods. After optional further processing of the new acyl chain, micafungin is purified to obtain the right quality and crystal form. Any suitable method, which may be known to the skilled person for purification of the desired cyclic peptide to the right quality and/or crystal form may be applied.

### Cyclic lipopeptide

The cyclic lipopeptide compound may be a cyclic lipopeptide such as daptomycin or amphomycin or a derivative thereof or a cyclic lipopeptide of the class of echinocandins. Preferably the cyclic lipopeptide is an echinocandin derivative. Echinocandins are compounds of general formula (**1**), wherein R is a hydrogen or an acyl group; R₁ is -H or -OH; R₂ is -H or -CH₃; R₃ is -H, -CH, -CH₂CONH₂ or -CH₂CH₂NH₂; R₄ is -H or -OH; R₅ is -OH, -OPO₃H₂, or -OSO₃H, R₆ is -H or -OSO₃H and R₇ is -H or CH₃.

Echinocandin derivatives may comprise any suitable enantiomer, and in a derivative R and R₁-₇ may have a different designation than described above.

In case a salt of a cyclic lipopeptide is applied in the method of the invention, the preferred salts are conventional nontoxic mono- or di-salts. These include metal salts such as alkali metal salts, alkaline earth metal salts, ammonium salts, salts with organic bases, organic acid addition salts, inorganic acid addition salts and salts with amino acids.

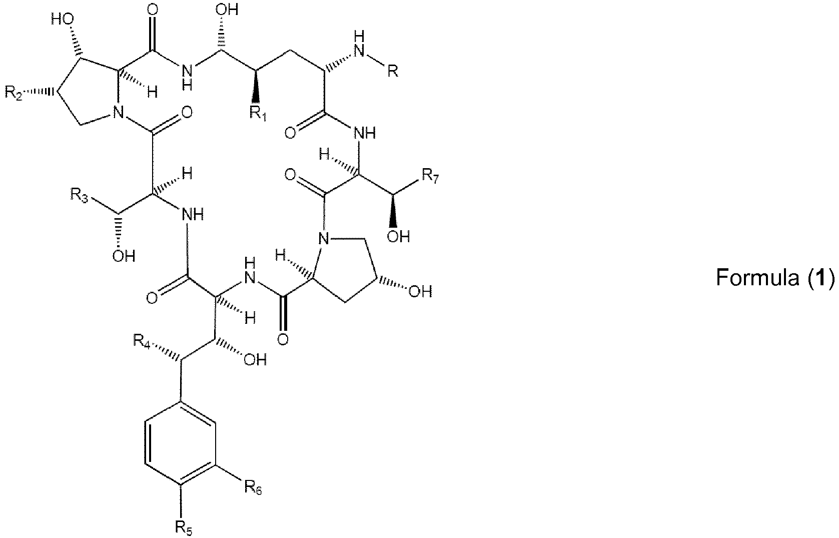

An acyl (IUPAC name: alkanoyl) group is usually derived from a carboxylic acid.

The term "acyl" in "acylamino" or "acyl" group may include aliphatic acyl, aromatic acyl, heterocyclic acyl, aryl-substituted aliphatic acyl, and heterocyclic-substituted aliphatic acyl derived from carboxylic acids, carbonic acids, carbamic acids, and sulfonic acids.

The term acyl may include lower alkanoyl, higher alkanoyl, lower alkenoyl, higher alkenoyl, higher alkoxycarbonyl, ar(lower)alkoxycarbonyl, lower alkylsulfonyl, arylsulfonyl, ar(lower)alkylsulfonyl, aroyl, lower alkyl, higher alkyl, higher alkoxy, higher alkenyloxy, carboxy and aryloxy, including derivatives of these with any suitable substituent(s).

The acyl group to be deacylated while using the method of the invention is preferably a higher alkanoyl group, such as a fatty acid acyl group. Such an acyl group may for example be a palmitoyl group or a myristate group. Pneumocandins for example have a hydrophobic dimethylmyristate tail connected via an amide bond to the alpha-amino group of its hydroxylated ornithine residue.

An echinocandin derivative suitable for use in the method of the invention may be selected from the group consisting of FR 901379, echinocandin B, aculeacin A and pneumocandin A, B or C.

An echinocandin that may be particularly suited for the method of the invention is Substance FR 901379 or analogs thereof with the general formula (**1**), in which R is acyl, R₁ is -OH, R₂ is -CH₃, R₃ is CH₂C(O)NH₂, R₄ is -OH, R₅ is OH, R₆ is -OSO₃H and R₇ is CH₃.

### Deacylation

In the present invention deacylation of a cyclic lipopeptide means that a side chain acylamino group of said cyclic lipopeptide is deacylated to an amino group.

For example, after deacylation of a cyclic lipopeptide of the class of echinocandins the deacylated compound is represented by the general formula (**2**), 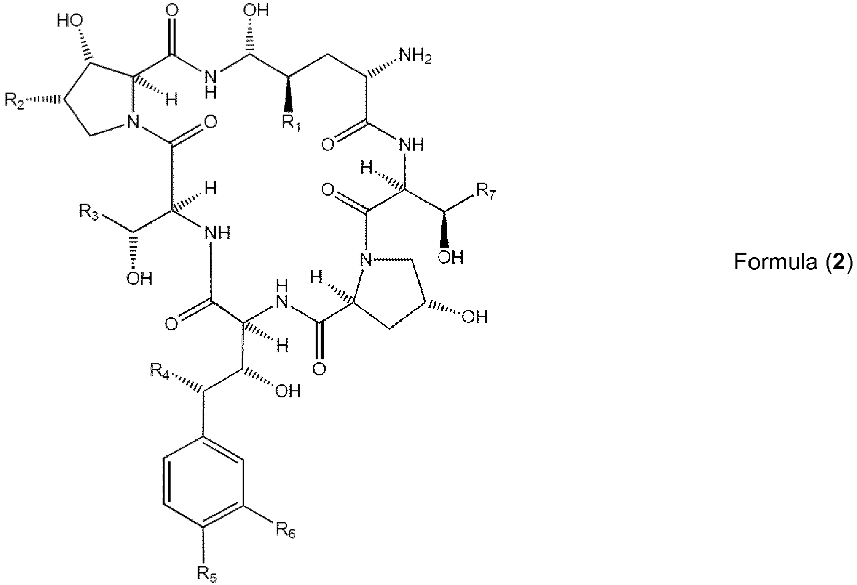 wherein R₁, R₂, R₃, R₄, R₅, R₆ and R₇ are as defined above for Formula (1).

The deacylation reaction may take place in any suitable medium. The pH is preferably near physiological, for instance from 5 to 10, preferably from 6 to 9, still more preferably from 6.5 to 8.5 such as about 8, and may, if desired, be adjusted by adding an amount of acid or base. The temperature at which deacylation is carried out is not very critical and may be from 0 to 50°C, preferably from 5 to 40°C. A suitable temperature may for example be a temperature between 20 and 30°C.

### Acylase

The acylase that is used to deacylate the lipid acyl moiety of a cyclic lipoprotein according to the method of the invention is an acylase produced by a microorganism belonging to the genus *Pseudomonas,* which includes Pseudomonads of the *Pseudomonas aeruginosa, Pseudomonas syringae*, *Pseudomonas putida, Pseudomonas pertucinogena, Pseudomonas fluorescens, Pseudomonas chlororaphis* and *Pseudomonas stutzeri* groups. The acylase applied in the method of the invention is preferably derived from *Pseudomonas aeruginosa.*

The term "derived from" is meant to include an acylase that originates from a *Pseudomonas* species as well as an artificial variant thereof. Preferably the acylase originates from a *Pseudomonas* species. However, it is also possible that a gene encoding such said acylase is made artificially on the basis of a known gene or protein sequence such as the sequences SEQ ID NO: 1 and SEQ ID NO: 2 disclosed herein. It is also possible that a gene encoding said acylase originating from *Pseudomonas* is artificially modified, which may lead to an artificial variant of said acylase which may be applicable in the method of the invention.

Surprisingly the inventors have found that an acylase from *Pseudomonas* is capable of removing the acyl chain of cyclic lipopeptides. The major advantage of using an acylase of *Pseudomonas* is that these acylases can be conveniently expressed in industrially preferred production hosts. In the method of the invention the acylase is obtained from an organism transformed with a vector encoding an acylase derived from *Pseudomonas.* Such an organism may be a bacterium or a yeast and is preferably selected from the group consisting of *Escherichia coli*, *Bacillus*, *Pichia pastoris* and *Kluyveromyces lactis.* In principle a non-pathogenic *Pseudomonas* host may also be a good host organism to produce an acylase in. Such a non-pathogenic *Pseudomonas* host may be for instance *Pseudomonas alcaligenes.* The acylase may as well be produced in fungi, such as *Aspergillus nidulans, Aspergillus niger* and *Aspergillus oryzae.*

An acylase that is particularly preferred is the protein PvdQ of *Pseudomonas aeruginosa* PA01, which has the amino acid sequence as set forth in SEQ ID NO:1 (Huang et al., 2003 Appl. Environ. Microbiol. 69: 5941-5949), or a functional equivalent homologue thereof. PvdQ was initially identified as an acyl homoserine lactone (AHL) acylase (Sio et al, Infect Immun. 2006 Mar; 74 (3): 1673-82). AHL acylases can degrade AHLs to produce fatty acid and homoserine lactone. This way AHL acylases can regulate quorum sensing activity in the bacteria. Surprisingly however, such an AHL acylase can also be applied to deacylate cyclic lipopeptides.

In *Pseudomonas* the AHL acylases are initially produced as a protein consisting of a signal peptide, an N-terminal alpha subunit or small subunit, a C-terminal beta subunit or large subunit and a so-called spacer peptide between the small alpha and the large beta subunit. Such a protein is referred to as preproprotein. The process of maturation of the preproprotein to an active enzyme comprises the removal of the signal peptide followed by an intramolecular cleavage of the remaining peptide chain into the small alpha and the large beta subunit and finally a part of the C-terminal amino acids of the small subunit (the spacer peptide) is removed. In the crude or purified enzyme preparation of the acylase used in the method, the acylase is preferably for a large part in its mature form.

The sequence set forth in SEQ ID NO: 1 represents a preproprotein and the gene with the sequence set forth in SEQ ID NO: 2 encodes this preproprotein. In case of SEQ ID NO: 1, the signal peptide is comprised in the amino acid residues 1-23. The size of the alpha subunit is approximately 18.5 kDa and the size of the beta subunit is approximately 60.4 kDa.

It will be understood that also functional derivatives of the acylase having the amino acid sequence of SEQ ID NO: 1 can be used in the method of the invention. The acylase used in the method of the invention therefore also encompasses derivatives and homologues of SEQ ID NO: 1. Protein sequences may be altered by substitutions, additions or deletions. Such protein sequences however may still encode functionally equivalent proteins or function-conservative variants. For example, several of the amino acids share similar properties. Arginine, lysine and histidine for example are positively charged at physiological pH. Aspartate and glutamate are negatively charged. Alanine, valine, isoleucine and leucine are small hydrophobic amino acids. Substitution of one or more amino acids within a protein sequence for another amino acid with the same properties therefore often leads to a functionally equivalent protein. For example, a substitution of valine for leucine within a protein sequence is usually not expected to influence protein functionality.

When the terms "homology", "sequence identity", "percent homology", or "percent identity" are used herein, they are used interchangeably. For the purpose of this invention, it is defined here that in order to determine the percent identity of two amino acid sequences or of two nucleic acid sequences, the complete sequences are aligned for optimal comparison purposes. In order to optimize the alignment between the two sequences gaps may be introduced in any of the two sequences that are compared. Such alignment is carried out over the full length of the sequences being compared. Alternatively, the alignment may be carried out over a shorter length, for example over about 20, about 50, about 100 or more nucleic acids/based or amino acids. The identity is the percentage of identical matches between the two sequences over the reported aligned region.

A comparison of sequences and determination of percent identity between two sequences can be accomplished using a mathematical algorithm. The skilled person will be aware of the fact that several different computer programs are available to align two sequences and determine the homology between two sequences (Kruskal, J. B. (1983) An overview of sequence comparison in D. Sankoff and J. B. Kruskal, (ed.), Time warps, string edits and macromolecules: the theory and practice of sequence comparison, pp. 1-44 Addison Wesley). The percent identity between two amino acid sequences can be determined using the Needleman and Wunsch algorithm for the alignment of two sequences. (Needleman, S. B. and Wunsch, C. D. (1970) J. Mol. Biol. 48, 443-453). The algorithm aligns amino acid sequences as well as nucleotide sequences. The Needleman-Wunsch algorithm has been implemented in the computer program NEEDLE. For the purpose of this invention the NEEDLE program from the EMBOSS package was used (version 2.8.0 or higher, EMBOSS: The European Molecular Biology Open Software Suite (2000) Rice, P. Longden, I. and Bleasby, A. Trends in Genetics 16, (6) 276-277, http://emboss.bioinformatics.nl/). For protein sequences, EBLOSUM62 is used for the substitution matrix. For nucleotide sequences, EDNAFULL is used. Other matrices can be specified. The optional parameters used for alignment of amino acid sequences are a gap-open penalty of 10 and a gap extension penalty of 0.5. The skilled person will appreciate that all these different parameters will yield slightly different results but that the overall percentage identity of two sequences is not significantly altered when using different algorithms.

### Global Homology Definition

The homology or identity is the percentage of identical matches between the two full sequences over the total aligned region including any gaps or extensions. The homology or identity between the two aligned sequences is calculated as follows: Number of corresponding positions in the alignment showing an identical amino acid in both sequences divided by the total length of the alignment including the gaps. The identity defined as herein can be obtained from NEEDLE and is labeled in the output of the program as "IDENTITY".

### Longest Identity Definition

The homology or identity between the two aligned sequences is calculated as follows: Number of corresponding positions in the alignment showing an identical amino acid in both sequences divided by the total length of the alignment after subtraction of the total number of gaps in the alignment. The identity defined as herein can be obtained from NEEDLE by using the NOBRIEF option and is labeled in the output of the program as "longest-identity". For purposes of the invention the level of identity (homology) between two sequences (amino acid or nucleotide) is calculated according to the definition of "longest-identity" as can be carried out by using the program NEEDLE.

The protein sequences of the present invention can further be used as a "query sequence" to perform a search against sequence databases, for example to identify other family members or related sequences. Such searches can be performed using the BLAST programs. Software for performing BLAST analyses is publicly available through the National Center for Biotechnology Information (http://www.ncbi.nlm.nih.gov). BLASTP is used for amino acid sequences and BLASTN for nucleotide sequences. The BLAST program uses as defaults:
- Cost to open gap: default = 5 for nucleotides/ 11 for proteins
- Cost to extend gap: default = 2 for nucleotides/ 1 for proteins
- Penalty for nucleotide mismatch: default = -3
- Reward for nucleotide match: default = 1
- Expect value: default = 10
- Word size: default = 11 for nucleotides/ 28 for megablast/ 3 for proteins

Furthermore the degree of local identity (homology) between the amino acid sequence query or nucleic acid sequence query and the retrieved homologous sequences is determined by the BLAST program. However only those sequence segments are compared that give a match above a certain threshold. Accordingly the program calculates the identity only for these matching segments. Therefore the identity calculated in this way is referred to as local identity.

The acylase encoded by abovementioned vector that is used in the method of the invention therefore is a protein which has an amino acid sequence as set forth in SEQ ID NO: 1, or an amino acid sequence with a sequence identity in the range between 50 and 100% thereto. Preferably said sequence identity is comprised between 60 and 100%, more preferably between 70 and 100%, more preferably between 80 and 100%, more preferably between 85 and 100%, more preferably between 90 and 100%, more preferably between 95 and 100%, most preferably between 98 and 100%.

The acylase used in the method of the invention preferably encompasses acylases with a sequence identity of between 90 and 100% with the acylase protein of *Pseudomonas aeruginosa* PA01 represented by SEQ ID NO: 1 (or at least the mature acylase thereof). Such acylases are preferably derived from *Pseudomonas aeruginosa* such as the acylases from *Pseudomonas aeruginosa* strains DK2, PAb1, M18, C3719, MPA01/P1, LESB58, PACS2, 39016, 2192, NCMG1179, PA7, PADK2_CF510.

### Acylase preparation

The acylase preparation that is used in the method of the invention may be a crude enzyme solution or a purified enzyme. The acylase preparation may also be composed of cells, either permeated or immobilized, that exhibit acylase activity or a homogenized cell suspension that exhibits acylase activity. Preferably, the acylase is produced in an industrially preferred production organism such as *Escherichia coli* in a fermenter. Cells are harvested and lysed with for instance a lysis buffer containing lysozyme to prepare the acylase preparation. Preferably lysed cells are then centrifuged and the soluble protein fraction containing the acylase is used as a crude enzyme preparation in the method of the invention.

In a preferred embodiment of the invention the crude or purified enzyme solution of acylase has been obtained from a production host such as *Escherichia coli.* For this purpose the gene encoding the acylase is isolated from the *Pseudomonas* genome, amplified and cloned into an expression vector. The expression vector can then be transformed into a host organism. Heterologous expression of a *Pseudomonas* acylase may be realized using various molecular biology techniques which are known to the skilled person.

Preferred alternative *Escherichia coli* host strains which can be used for industrial production of protein comprise *Escherichia coli* DH10B, *Escherichia coli* BL21, K12 strain RV308, *Escherichia coli* DH5, *Escherichia coli* JM109, *Escherichia coli* XL-1 and derivatives of these strains. Such derivatives may comprise specific knock-outs or mutations which are beneficial for recombinant protein production and/or the use of certain desired markers and/or promoters. Various examples can be found at http://wolfson.huji.ac.il/expression/bac-strains-prot-exp.html.

If necessary a gene encoding said acylase can be synthesized in order to be codon optimized for its host organism. Different organisms often show different preferences for one of the several codons that encode the same amino acid. Such a preference occurs when a greater frequency of a particular codon is found than expected by chance. Codon preference is thought to play a role in particular in fast-growing bacteria such as *Escherichia coli,* wherein the genomic tRNA pool reflects the codon preference. If high amounts of proteins should be expressed in such an organism, optimal codons could help to achieve faster translation rates and thus more protein production. Codon optimization could in particular be relevant in case of heterologous expression as is the case in the present invention, wherein a *Pseudomonas* protein is expressed in a preferred industrial fermentation organism such as *Escherichia coli.*

Another way to optimize protein production in a preferred industrial fermentation strain such as *Escherichia coli* may be to use an alternative signal peptide. As mentioned above, proteins such as the AHL acylase of *Pseudomonas* are initially synthesized as a preproprotein. The nucleotide sequence coding for the AHL acylase without a signal peptide can be operably linked to a nucleotide sequence coding for a signal peptide from another species which also permits secretion of the preproprotein over the cytoplasmic membrane into the periplasm. Exchanging the signal peptide of a given protein for another might favor secretion of the protein in an industrially preferred production host organism and thus help to achieve more and faster protein production.

Further variations in expression constructs may be in promoters expressing the genes, way of induction or in copy number (low, medium, or high) of the plasmids.

Various expression constructs and expression hosts can be used. In preferred expression constructs the genes are expressed by either an inducible or a constitutive promoter using a low, medium, or high copy expression vector.

### EXAMPLES

### Example 1

### Deacylation of a cyclic lipopeptide

A detailed description of how the method of the invention may be performed is disclosed in the following example. Although this example describes the deacylation of pneumocandin B₀ and FR 901379 the skilled person will acknowledge that the methods presented in this example will be well applicable for other cyclic lipopeptides.

### Gene cloning and expression hosts

### Expression using an Escherichia coli expression matrix

SEQ ID NO: 2 represents the DNA sequence of the gene encoding the wild-type acylase from *Pseudomonas aeruginosa* PA01 with its native signal peptide. The amino acid sequence of the protein encoded by this DNA sequence is set forth in SEQ ID NO: 1. The signal peptide represents residues 1-23 of SEQ ID NO: 1. This gene was re-cloned from the original vector used by the synthesis vendor (DNA2.0) into a pTAC low copy expression vector behind a wild-type IPTG inducible tac promoter using specific restriction sites. Subsequently the expression construct was transformed into an *Escherichia coli* DH10B expression host. Recombinant colonies were selected by antibiotic resistance (kanamycin) and analyzed by restriction digestion. Bacteria were grown *in duplo* in 24 wells microtiter plates using conditions as follows.

### 24 wells microtiter plate fermentations

Individual colonies were grown overnight in 3 ml 2xTY medium (in presence and absence of 100 µg/ml kanamycin) in 24 wells microtiter plates at 30°C and 550 rpm. Subsequently the fermentation broth was diluted 1:100 in 24 wells microtiter plates at 30°C and 550 rpm using Slow Glucose Release Medium (in presence or absence of 100 µg/ml kanamycin). Cultures were grown for another 48 hours after adding 50 µM IPTG. After freezing and thawing the cell pellets were resuspended in lysis buffer and centrifuged. The supernatant (soluble fraction) was used for qualitative SDS-PAGE analysis using NuPAGE MOPS buffer and SeeBlue plus2 marker. Proteins were visualized by staining with Instant Blue.

### Qualitative SDS-PAGE

The expression of the acylase gene was analyzed using qualitative SDS-PAGE. For qualitative SDS-PAGE the cell pellets were collected by centrifugation at 14,000 rpm for 1 minute in a bench-top Eppendorf centrifuge and were dissolved in lysis buffer (100 mM Tris/HCI, 0.1 mg/ml DNAse, 2.0 mg/ml Lysozyme, 25 µM MgSO₄) and incubated for 30 minutes at 37°C. Samples were fractionated by centrifugation and lysed cell pellets were centrifuged for 1 minute at 14,000 rpm in a bench-top Eppendorf centrifuge and the soluble protein fraction was transferred into a fresh mini tube. Pellets were dissolved in an equal volume of the Phosphate Buffered Saline (PBS) buffer. 6.5 µl of the supernatant and 6.5 µl of the pellet fraction were used for SDS-PAGE analysis.

### Shake flask fermentations

Bacteria were grown using conditions as described in the previous paragraph. Final growth is performed in a 100 ml shake-flask, with foam plug, containing 10 mL medium, at 30°C and 280 rpm.

### Sample pre-treatment for activity assay

For the activity assay the bacteria were grown in Slow Glucose Release Medium (in presence or absence of 100 µg/ml kanamycin) in 100 mL Shake-Flask. Selected strains were grown using conditions as described above. Cell pellets were collected by centrifugation at 14,000 rpm for 1 minute in a bench-top Eppendorf centrifuge and were dissolved in lysis buffer (100 mM Tris/HCl, 0.1 mg/ml DNAse, 2.0 mg/ml Lysozyme, 25 µM MgSO₄) and incubated for 30 minutes at 37°C. Samples were fractionated by centrifugation and lysed cell pellets were centrifuged for 1 minute at 14,000 rpm in a bench-top Eppendorf centrifuge and the soluble protein fraction was transferred into a fresh mini tube.

### Deacylation of pneumocandin B₀

After shake-flask fermentation of *Escherichia coli* expressing the *Pseudomonas aeruginosa* acylase gene the ability of the produced acylase to deacylate pneumocandin B₀ was determined. The enzyme reactions were performed according to the following conditions:
4 ml 100 mM di-potassium hydrogen phosphate pH 8.0;
Add 0.5 ml 1 mg/ml pneumocandin B₀ (dissolved in MeOH);
Add 0.5 ml sample (soluble protein fraction containing the acylase).

| | |
|---|---|
| Reaction temperature: | 40°C |
| Reaction time: | 1 hour |

The enzyme reaction was stopped by dilution of the reaction mixture with 2% phosphoric acid 1:1 (v/v). Subsequently the reaction mixture was applied to a UPLC chromatography system coupled to a mass spectrometer for the determination of pneumocandin B₀ (substrate), deacylated pneumocandin B₀ (product) and its hydrolysis product (deacylated pneumocandin B₀-H₂O).

### Deacylation of FR 901379

After shake-flask fermentation of *Escherichia coli* expressing the *Pseudomonas aeruginosa* acylase gene the ability of the acylase to deacylate FR 901379 was determined. The enzyme reactions were performed according to the following conditions:
4 ml 100 mM di-potassium hydrogen phosphate pH 8.0;
Add 0.5 ml 1 mg/ml FR 901379 (dissolved in MeOH);
Add 0.5 ml sample (soluble protein fraction containing the acylase)

| | |
|---|---|
| Reaction temperature: | 40°C |
| Reaction time: | 1 hour |

The enzyme reaction was stopped by dilution of the reaction mixture with 2% phosphoric acid 1:1 (v/v). Subsequently the reaction mixture was applied to a UPLC chromatography system coupled to a mass spectrometer for the determination of FR 901379 (substrate), deacylated FR 901379 (product) and its hydrolysis product (deacylated FR 901379-H₂O).

### Method of analysis

Reaction products were analyzed using UPLC connected to a mass spectrometer for detection.

| | |
|---|---|
| Column: | Waters UPLC HSS T3 1.8um 2.1 * 100 mm I.D 1.7 um |
| Mobile phase A | 50 mM Ammonium acetate |
| Mobile phase B | 75% acetonitrile |
| Column temp. | 40°C |
| Flow | 0.35 ml/min |

Gradient for pneumocandin B₀:

| | | | | | | |
|---|---|---|---|---|---|---|
| Time (min) | 0 | 3 | 5 | 6 | 5.2 | 8 |
| %A | 98 | 90 | 10 | 10 | 98 | 98 |
| %B | 2 | 10 | 90 | 90 | 2 | 2 |

Gradient for FR 901379:

| | | | | | | |
|---|---|---|---|---|---|---|
| Time (min) | 0 | 3 | 5 | 6 | 5.2 | 8 |
| %A | 98 | 75 | 10 | 10 | 98 | 98 |
| %B | 2 | 25 | 90 | 90 | 2 | 2 |

Mass spectrometer Instrument Parameters - Function 1:

| | |
|---|---|
| Polarity | ES+ |
| Calibration | Static 1 |
| Capillary (kV) | 3.00 |
| Cone (V) | 40.00 |
| Extractor (V) | 1.00 |
| RF Lens (V) | 1.0 |
| Source Temperature (°C) | 120 |
| Desolvation Temperature (°C) | 350 |
| Cone Gas Flow (L/h) | 100 |
| Desolvation Gas Flow (L/h) | 500 496 |
| LM 1 Resolution | 15.0 |
| HM 1 Resolution | 15.0 |
| Ion Energy 1 | 0.5 |
| Entrance | 40 |
| Collision | 0 |
| Exit | 40 |
| LM 2 Resolution | 15.0 |
| HM 2 Resolution | 15.0 |
| Ion Energy 2 | 0.5 |
| Multiplier (V) | 700 |
| Syringe Pump Flow (uL/min) | 20.0 |
| Gas Cell Pirani Pressure (mbar) | <1e-4 mbar |

Since neither standards of deacylated pneumocandin B₀ and deacylated FR 901379, nor standards of the corresponding hydrolysis products or labeled internal standards of pneumocandin B₀ and FR 901379 were available, only qualitative analysis of these components was performed. Therefore the results are only estimation and they are not corrected for any matrix effect (ion suppression in mass spectrometry, etc.).

### Results

### Expression of the Pseudomonas aeruginosa acylases

SDS PAGE analysis showed that the acylase from *Pseudomonas aeruginosa* was expressed in the *Escherichia coli* DH10B strain. The correct sizes of the alpha subunit (18.5 kDa) and beta subunit (60.4 kDa) were detected in the soluble fraction.

**Table 1: Peak areas of deacylated pneumocandin B₀, its hydrolysis product and pneumocandin B₀**

| Sample name | deacylated pneumocandin B₀ | hydrolysis product | pneumocandin B ₀ |
|---|---|---|---|
| Blank | | | 123881 |
| *Escherichia coli* empty strain | | | 139014 |
| *Escherichia coli* + pTAC_SEQ ID NO: 2 | 1376 | 2653 | 121497 |

After shake-flask fermentation, lysis and recovery of the soluble fraction as described, the activity of the supernatant containing the acylase of the invention was tested on pneumocandin B₀. Table 1 shows the activity of different samples after shake-flask fermentation on pneumocandin B₀. In this table the peak area of deacylated pneumocandin B₀, its hydrolysis product and pneumocandin B₀ are presented. As blank sample (Blank), a reaction without enzyme (or sample) is performed. In addition, to correct for any possible background from the expression host an empty strain (DH10B) was also included. In both the blank sample and in the sample to which the cell free extract of the empty production strain was added only the pneumocandin B₀ peak was observed. When incubated with the cell free extract of the cells expressing the acylase (DH10B + pTAC_SEQ ID NO: 2) both deacylated pneumocandin B₀ and its hydrolysis product are observed. The presence of these components shows that the acylase which was characterized as an acyl homoserine lactone acylase does also show deacylating activity for the cyclic lipopeptide pneumocandin B₀ resulting in the desired deacylated pneumocandin B₀ and the free acyl side chain.

In separate experiment the shake-flask fermentation, lysis and recovery of the soluble fraction were repeated and the activity of the supernatant containing the acylase of the invention was tested on FR 901379. Table 2 shows the activity of different samples after shake-flask fermentation on FR 901379.

**Table 2: Peak areas of deacylated FR 901379, its hydrolysis product and FR 901379**

| Sample name | deacylated FR 901379 | hydrolysi s product | FR 901379 |
|---|---|---|---|
| Blank | | | 109857 |
| *Escherichia coli* empty strain | | | 194490 |
| *Escherichia coli* + pTAC_SEQ ID NO: 2 | 4808 | 605 | 51673 |

After incubation of FR 901379 with the cell free extract of an *Escherichia coli* fermentation producing the *Pseudomonas aeruginosa* acylase both deacylated FR 901379 and its hydrolysis product are observed. The presence of these components shows that the acylase which was shown to deacylate pneumocandin B₀ is also able to deacylate the cyclic lipopeptide FR 901379 resulting in the desired deacylated FR 901379 and the free acyl side chain.

### Conclusions

The acylase from *Pseudomonas aeruginosa* shows activity towards cyclic lipopeptides. A vector encoding the amino acid sequence as set forth in SEQ ID NO: 1 is therefore suitable for use in the method of the invention. This example shows that by using a *Pseudomonas* acylase in a method of producing cyclic peptides from cyclic lipopeptides it becomes possible to produce a cyclic peptide from a cyclic lipopeptide in an efficient way. The method of the invention makes it possible to produce a cyclic peptide from a cyclic lipopeptide in an efficient way, because the *Pseudomonas* acylase can be produced in an industrially preferred production organism (in this example *Escherichia coli*).

In case of pneumocandin B₀ the acylase cleaves off a 10,12-dimethyl-myristoyl acyl moiety. Another cyclic lipopeptide containing a 10,12-dimethyl-myristoyl acyl moiety is sporiofungin A. Given the deacylation of pneumocandin B₀ it is very likely the acylase will also deacylate sporiofungin A. In case of FR 901379 the acylase cleaves off a palmitoyl acyl moiety. Aculeacin A, FR 209602 and cryptocandin also carry the palmitoyl acyl moiety and therefore are also suitable to be deacylated by the acylase.

### Example 2

### Conserved residues of the acylase of the invention

In the following example the active site residues of the acylase used in the method of the invention were determined by computational studies.

For this purpose the 3D structure of the mature quorum quenching n-acyl homoserine lactone acylase from *Pseudomonas aeruginosa* PA01 (PvdQ, represented by amino acid residues 24 to 762 of SEQ ID NO: 1) as determined by M. Bokhove et al. (2010), Proc Natl Acad Sci USA 107,686-691 was taken as a model. The atomic coordinates are available from the Protein Data Bank by accession code 2WYB. The inventors used the molecular modeling package Maestro (Schrödinger) to visualize the 3D structure of 2WYB which contains a covalently bound dodecanoic acid in the active site. The dodecanoic acid was used to map the active site of the acylase in order to determine the amino acids that contribute to its substrate specificity which surprisingly includes the deacylation of cyclic lipopeptides.

Residues in the active site can be divided in so-called first shell amino acids and second shell amino acids. The first shell amino acids are those amino acids which interact directly with the dodecanoic acid via electrostatic interactions, hydrogen bonding, hydrophobic interactions and/or van der Waals forces. Apart from the first shell amino acids which interact directly with the dodecanoic acid also the second shell amino acids are considered. The second shell residues are identified by measuring which amino acids have at least one atom within a sphere of 7 Angstroms around a first shell amino acid.

The inventors used the dodecanoic acid as a guide to determine the docking of cyclic lipopeptides (*e*.*g*. pneumocandin B₀, FR 901379) into the active site. The resulting complexes can be used to extend the mapping of the active site towards the cyclic peptide moiety of the substrate in the same way as described for the dodecanoic acid. In order to generate a 3D model for homologous amino acid sequences originating from a *Pseudomonas* species or synthetic variants thereof for which no experimental 3D structure is available comparative modeling using the modeling software package Prime (Schrodinger) was applied. It should be understood that alternative software tools with similar functionality are available for comparative modeling *e.g.* Yasara, Modeler, Swiss-Model, etc., and will lead to the same results. By superimposing the active site which was mapped previously according to the procedure as described above onto the 3D model of the homologous protein originating from a *Pseudomonas* species or an artificial variant thereof, it can be established whether the given homologous protein can be expected to also exhibit deacylation activity for cyclic lipopeptides. Homologous proteins in this aspect originate from *Pseudomonas* species and show at least 50% sequence identity (such as 60, 70, 80, 85, 90, 95, or 98 % sequence identity) with SEQ ID NO: 1. The person skilled in the art will understand that such homologous proteins may be derived from original *Pseudomonas* wild type genes or from artificial variants thereof.

Comparative modeling of the active site performed as described above revealed that an acylase usable in the method of the invention typically contains the following sequence motifs:
i) D-X₄-[V or T]-X₂-L-[L or M]-X₃-G;
ii) [L or I]-P-G-L-P-X₂-N;
iii) R-V-L-X₂-W; and
iv) H-T-V-D-X₃-H,
wherein Xₙ is a sequence of unspecified amino acid residues of length "n". For instance, X₄ means X-X-X-X, which indicates that four sequential positions may contain any amino acid residue. Amino acid residues within brackets, like [V or T], indicates that the amino acid residue at this position may be V or T.

Motif i) corresponds, as determined by protein sequence alignment, to the amino acid residues at positions 161-174 of SEQ ID NO: 1, wherein the amino acid residues V or T at position 166, L at position 169 and L or M at position 170 are first shell residues contacting the substrate.

Motif ii) corresponds, as determined by protein sequence alignment, to the amino acid residues at positions 266-273 of SEQ ID NO: 1, wherein the amino acid residues L or I at position 266, L at position 269 and N at position 273 are first shell residues contacting the substrate.

Motif iii) corresponds, as determined by protein sequence alignment, to the amino acid residues at positions 373-378 of SEQ ID NO: 1, wherein the amino acid residues V at position 374, L at position 375 and W at position 378 are first shell residues contacting the substrate.

Motif iv) corresponds, as determined by protein sequence alignment, to the amino acid residues at positions 284-291 of SEQ ID NO: 1, wherein the amino acid residues H at position 284, T at position 285 and V at position 286 are first shell residues contacting the substrate.

For these reasons, the vector used in the method of the invention preferably encodes an acylase that has an amino acid sequence that has between 50 and 100 % (such as between 50, 60, 70, 80, 85, 90, 95 or 99 and 100%) sequence identity with respect to the amino acid sequence set forth in SEQ ID NO: 1, wherein the amino acid sequence of said acylase contains the following sequence motifs:
i) D-X₄-[V or T]-X₂-L-[L or M]-X₃-G (SEQ ID NO: 3);
ii) [L or I]-P-G-L-P-X₂-N (SEQ ID NO: 4);
iii) R-V-L-X₂-W (SEQ ID NO: 5); and
iv) H-T-V-D-X₃-H (SEQ ID NO: 6),
wherein Xₙ is a sequence of unspecified amino acid residues of length "n".

## Claims

1. A method for producing a cyclic peptide compound or a salt thereof which comprises contacting a cyclic lipopeptide compound or a salt thereof with an acylase which has an amino acid sequence as set forth in SEQ ID NO: 1 or an amino acid sequence with a sequence identity in the range between 90 to 100% thereto.

2. The method according to claim 1, wherein the amino acid sequence of said acylase contains the following sequence motifs:
i) D-X₄-[V or T]-X₂-L-[L or M]-X₃-G (SEQ ID NO: 3);
ii) [L or I]-P-G-L-P-X₂-N (SEQ ID NO: 4);
iii) R-V-L-X₂-W (SEQ ID NO: 5); and
iv) H-T-V-D-X₃-H (SEQ ID NO: 6),
wherein Xₙ is a sequence of unspecified amino acid residues of length "n".

3. The method according to any one of claims 1-2, comprising an additional step of reacylating said cyclic peptide resulting in a reacylated cyclic peptide.

4. The method according to claim 3, wherein the acyl chain of said reacylated cyclic peptide is further processed resulting in a further processed reacylated cyclic peptide.

5. The method according to any of the claims 1-4, wherein said acylase is obtained from an organism transformed with a vector encoding an acylase derived from *Pseudomonas.*

6. The method according to claim 5, wherein said organism is selected from the group consisting of *Escherichia coli*, *Bacillus, Pseudomonas alcaligenes, Pichia pastoris* and *Kluyveromyces lactis.*

7. The method according to any of the claims 5-6, wherein the vector encodes the amino acid sequence as set forth in SEQ ID NO: 1.

8. The method according to any of the claims 1-7, wherein said acylase is derived from *Pseudomonas aeruginosa.*

9. The method according to any of the claims 1-8, wherein said cyclic lipopeptide is an echinocandin derivative.

10. The method according to claim 9, wherein the echinocandin derivative is selected from the group consisting of aculeacin A, cryptocandin, echinocandin B, FR 209602, FR 901379, pneumocandins A, B and C and sporiofungin.

11. The method according to any of the claims 3-10, wherein said reacylated cyclic peptide or said further processed reacylated cyclic peptide is micafungin or a salt thereof.

## Patentansprüche

1. Verfahren zum Herstellen einer zyklischen Peptidverbindung oder eines Salzes davon, umfassend das Kontaktieren einer zyklischen Lipopeptidverbindung oder eines Salzes davon mit einer Acylase, die eine Aminosäurensequenz wie in SEQ ID NO: 1 dargelegt oder eine Aminosäurensequenz mit einer Sequenzidentität im Bereich von 90 bis 100 % dazu aufweist.

2. Verfahren nach Anspruch 1, wobei die Aminosäurensequenz der Acylase die folgenden Sequenzmotive enthält:
i) D-X₄-[V oder T]-X₂-L-[L oder M]-X₃-G (SEQ ID NO: 3);
ii) [L oder I]-P-G-L-P-X₂-N (SEQ ID NO: 4);
iii) R-V-L-X₂-W (SEQ ID NO: 5); und
iv) H-T-V-D-X₃-H (SEQ ID NO: 6)
wobei Xₙ eine Sequenz unbestimmter Aminosäurereste der Länge "n" ist.

3. Verfahren nach einem der Ansprüche 1-2, umfassend einen zusätzlichen Schritt des Reacylierens des zyklischen Peptids, was zu einem reacylierten zyklischen Peptid führt.

4. Verfahren nach Anspruch 3, wobei die Acylkette des reacylierten zyklischen Peptids weiter behandelt wird, was zu einem weiter behandelten reacylierten zyklischen Peptid führt.

5. Verfahren nach einem der Ansprüche 1-4, wobei die Acylase aus einem Organismus erhalten wird, der mit einem Vektor transformiert wird, der eine Acylase abgeleitet von *Pseudomonas* kodiert.

6. Verfahren nach Anspruch 5, wobei der Organismus aus der Gruppe bestehend aus *Escherichia coli, Bacillus, Pseudomonas alcaligenes, Pichia pastoris* und *Kluyveromyces lactis* ausgewählt ist.

7. Verfahren nach einem der Ansprüche 5-6, wobei der Vektor die Aminosäurensequenz wie in SEQ ID NO: 1 dargelegt kodiert.

8. Verfahren nach einem der Ansprüche 1-7, wobei die Acylase von *Pseudomonas aeruginosa* abgeleitet ist.

9. Verfahren nach einem der Ansprüche 1-8, wobei das zyklische Lipopeptid ein Echinocandin-Derivat ist.

10. Verfahren nach Anspruch 3, wobei das Echinocandin-Derivat aus der Gruppe bestehend aus Aculeacin A, Cryptocandin, Echinocandin B, FR 209602, FR 901379, Pneumocandinen A, B und C und Sporiofungin ausgewählt ist.

11. Verfahren nach einem der Ansprüche 3-10, wobei das reacylierte zyklische Peptid oder das weiter behandelte reacylierte zyklische Peptid Micafungin oder ein Salz davon ist.

## Revendications

1. Procédé pour produire un composé de peptide cyclique ou un sel de celui-ci, qui comprend la mise en contact d'un composé de lipopeptide cyclique ou d'un sel de celui-ci avec une acylase qui a une séquence d'acides aminés telle que présentée dans SEQ ID N0: 1 ou une séquence d'acides aminés ayant une identité de séquence dans la gamme entre 90 à 100% avec celle-ci.

2. Procédé selon la revendication 1, dans lequel la séquence d'acides aminés de ladite acylase contient les motifs de séquence suivantes :
i) D-X₄-[V ou T]-X₂-L-[L ou M]-X₃-G (SEQ ID NO: 3) ;
ii) [L ou I]-P-G-L-P-X₂-N (SEQ ID NO: 4) ;
iii) R-V-L-X₂-W (SEQ ID NO: 5) ; et
iv) H-T-V-D-X₃-H (SEQ ID NO: 6),
dans lesquels Xₙ est une séquence de restes d'acides aminés non spécifiés de longueur "n".

3. Procédé selon l'une quelconque des revendications 1-2, comprenant une étape supplémentaire consistant à réacyler ledit peptide cyclique, conduisant à un peptide cyclique réacylé.

4. Procédé selon la revendication 3, dans lequel la chaîne acyle dudit peptide cyclique réacylé est traitée supplémentairement, conduisant à un peptide cyclique réacylé traité supplémentairement.

5. Procédé selon l'une quelconque des revendications 1 à 4, dans lequel ladite acylase est obtenue à partir d'un organisme transformé avec un vecteur codant une acylase issue de *Pseudomonas.*

6. Procédé selon la revendication 5, dans lequel ledit organisme est choisi dans le groupe constitué par *Escherichia coli, Bacillus, Pseudomonas alcaligenes, Pichia pastoris* et *Kluyveromyces lactis.*

7. Procédé selon l'une quelconque des revendications 5-6, dans lequel le vecteur code la séquence d' acides aminés telle que présentée dans SEQ ID NO: 1.

8. Procédé selon l'une quelconque des revendications 1 à 7, dans lequel ladite acylase est issue de *Pseudomonas aeruginosa.*

9. Procédé selon l'une quelconque des revendications 1 à 8, dans lequel ledit lipopeptide cyclique est un dérivé d'échinocandine.

10. Procédé selon la revendication 9, dans lequel le dérivé d'échinocandine est choisi dans le groupe constitué par l'aculéacine A, une cryptocandine, l'échinocandine B, FR 209602, FR 901379, les pneumocandines A, B et C et une sporiofungine.

11. Procédé selon l'une quelconque des revendications 3 à 10, dans lequel ledit peptide cyclique réacylé ou ledit peptide cyclique réacylé traité supplémentairement est une micafungine ou un sel de celle-ci.
